(19) **Europäisches Patentamt**

**European Patent Office**

**Office européen des brevets**

(11) **EP 0 865 262 B1**

(12) **EUROPEAN PATENT SPECIFICATION**

(45) Date of publication and mention
of the grant of the patent:
**09.01.2002 Bulletin 2002/02**

(51) Int Cl.⁷: **A61F 2/68**
// A61F4/00

(21) Application number: **96935608.8**

(22) Date of filing: **22.10.1996**

(86) International application number:
**PCT/NO96/00246**

(87) International publication number:
**WO 97/15249 (01.05.1997 Gazette 1997/19)**

(54) **DEVICE FOR CONTROL OF PROSTHESES AND OTHER ASSISTANCE DEVICES**

VORRICHTUNG ZUR KONTROLLE VON PROTHESEN UND ANDERER HILFSMITTEL

APPAREIL PERMETTAN L'ACTIVATION D'UNE PROTHESE OU DE TOUT AUTRE APPAREIL D'ASSISTANCE

(84) Designated Contracting States:
**AT BE CH DE DK ES FI FR GB GR IE IT LI LU MC
NL PT SE**
Designated Extension States:
**AL LT LV RO SI**

(30) Priority: **23.10.1995 NO 954221**

(43) Date of publication of application:
**23.09.1998 Bulletin 1998/39**

(73) Proprietor: **Leiv Eiriksson Nyfotek AS
7005 Trondheim (NO)**

(72) Inventors:
• **STAVDAHL, Öyvind
N-7014 Trondheim (NO)**
• **GRÖNNINGSAETER, Aage
N-7039 Trondheim (NO)**

(74) Representative: **Olsson, Jan et al
Bjerkéns Patentbyra KB P.O.Box 1274
801 37 Gävle (SE)**

(56) References cited:
**US-A- 4 074 367        US-A- 4 571 750
US-A- 4 770 662**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 865 262 B1

**Description**

The present invention describes a device for control of prostheses and other assistance devices, according to the introductory part of claim 1.

The term "prosthesis" primarily refers to an artificial hand, arm, foot, leg or the like, with one or more motorized joints. The term can be generalized to assistance devices, so that it refers also to other mechanical devices, utilized by a handicapped person to compensate for a missing or defective limb or part of a limb, in order to replace at least parts of the missing limb's function. Examples of these are active or actively lockable orthoses, wheelchairs and other orthopedic or assistive devices. Furthermore, the term "assistance device" can refer to all controllable technical systems utilized by handicapped and/or healthy persons, such as robots for telemanipulation, miniature manipulators for endoscopic operations, as well as machinery, tools and vehicles in general; program systems, computers and the like.

The term "prosthetic state" refers to a mechanical or geometrical state in the prosthesis, such as an angular or linear position, angular or linear velocity, torque/force, etc., or a combination of such figures.

In the following, the term "muscle" refers to a muscle in the physiological sense, as well as to other tissue and/or bones/joints, which can be deformed or moved under direct or indirect control by the person's central nervous system, or a combination of these. "Muscle contraction" refers to the resulting mechanical or geometrical changes in a muscle. The term "motor intention" refers to the specific function that the user of the present invention desires to activate. In the case of a prosthesis in the usual sense, "motor intention" refers to the motor movement or movement pattern that the user wants the prosthesis to perform.

Prosthesis control based on myoelectric signals is known. One example of this can be found in EP patent publication 0 421 780. US patent publication 4 571 750 shows exploitation of myoacoustic signals for the same purpose. These known methods use, among other things, the amplitude of the detected signal as a basis for controlling the prosthesis. Variations in skin moisture as well as electric and acoustic noise from the environment frequently cause undesired variations in the signal amplitude, and thereby directly influence the control of the prosthesis. Furthermore, myoelectric and myoacoustic signals measured on the skin surface express a weighted sum of the activities of all muscles in the area where the detecting transducer is placed, but it is difficult to discern between the contributions of the individual muscles.

It is therefore an object of the present invention to provide a device for control of prostheses and other assistance devices, which has a greater tolerance with regards to disturbances than is the case in previously known solutions, so the person's motor intention can be estimated more easily. Moreover, it is an object of the present invention to provide a device for the control of prostheses and the like, which allows for identification of muscle contraction in multiple subcutaneous muscles simultaneously, so that the person's motor intention can be estimated in greater detail and in a greater number of degrees of freedom.

The object of the invention is achieved by a device decribed by the characterizing part of Claim 1. Additional characteristics are described in the related dependent claims.

In the following, the invention will be explained in further detail by way of examples of embodiments and with reference to the accompanying drawings, where

Fig. 1a shows an axial cross section of a body part with a muscle in a contracted state,
Fig. 1b shows an axial cross section of a body part with a muscle in a relaxed state,
Fig. 2 shows a cross section of a body part with different tissue structures, where dotted lines illustrate how geometric information can be collected from a number of muscles by use of ultrasound measurements in a number of directions, according to the present invention,
Fig. 3 shows a cross section of a body part with different tissue structures, where external transducers are arranged according to the present invention,
Fig. 4 shows a cross section of a body part with different tissue structures, where external and implanted transducers and reflectors are arranged in accordance with the present invention, and
Fig. 5 shows a principle sketch of an on/off-control system according to the present invention.

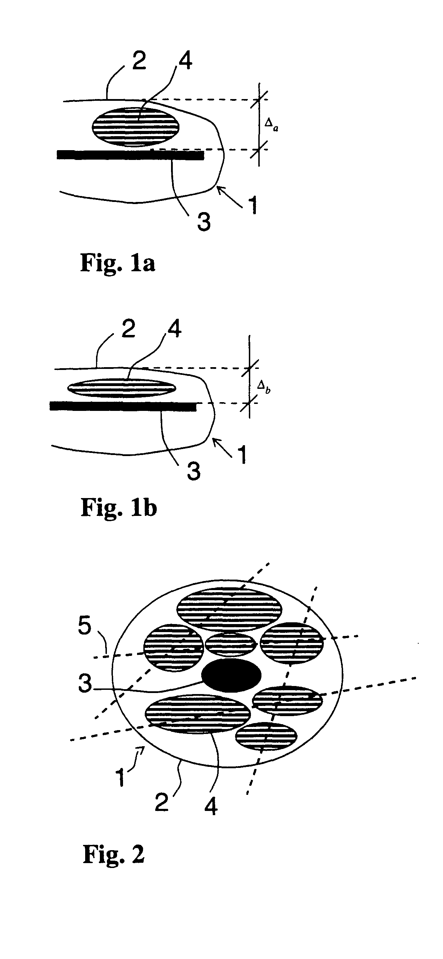

Fig. 1a illustrates a representation of a body part 1 with a skin surface 2, a bone 3 and a muscle 4. The muscle 4 is shown in a contracted (shortened) state, and the distance from the skin surface 2 to the bone 3 is denoted $\Delta_a$. Fig. 1b shows the body part 1 with the muscle 4 in a relaxed state, with the distance from the skin surface 2 to the bone 3 denoted $\Delta_b$. The muscle's 4 geometry is seen to change upon contraction, so that the distance $\Delta_a$ from the skin surface 2 to the bone 3 during muscle contraction is greater than the distance $\Delta_b$ during muscle relaxation. The degree of muscle contraction in the case illustrated in Fig. 1a is given by the equation $(\Delta_a - \Delta_b)$.

According to the invention, measurements of the relaxed and contracted states of the muscles are made by transmitting an ultrasound pulse into the tissue and then receiving the echo from the tissue structures. In fact the different tissue structures are arranged in several layers. This is illustrated in Fig. 2. Here, a number of muscles 4 are distributed in the body part 1 around the bone 3. To measure the state of all muscles 4, it is sufficient to make ultrasound shots 5 in a few directions; in the present example ultrasound shots 5 are made in four directions in order to observe seven muscles 4. When performing one-dimensional data collection (i.e. using only one beam), the only component of the muscle deformation that can be observed is that coinciding with the direction of the beam. By performing two-dimensional data collection i.e. in a plane, the muscle deformation components in this plane (two dimensions) can be observed. By performing three-dimensional data collection i.e. in a volume, the muscle deformation components in all three dimensions can be observed.

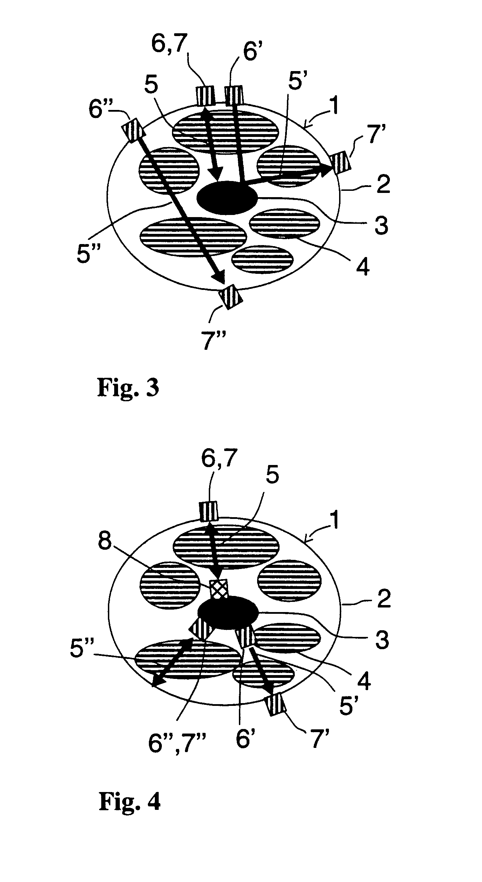

The practical implementation of the present invention can be achieved by the use of ultrasound transducers which are in acoustic contact with the skin surface, or alternativeley implanted into the body. Fig. 3 shows three possible implementations of the invention, where a combined transmitting/receiving transducer 6,7 as well as two transmitting transducers 6', 6" and two receiving transducers 7', 7" are placed on the body part 1, in contact with the skin surface 2. Ultrasound pulses 5 are transmitted into the body part 1 by the combined transmitting/receiving transducer 6,7, and echo from subcutaneous tissue structures are received by the same transducer 6,7. In Fig. 3 only the echo from the bone 3 is illustrated, while echo from other tissue structures will also provide information about muscle contractions in the relevant region. The transmitting transducer 6' transmits ultrasound pulses towards the bone 3, and the echo 5' is received by the receiving transducer 7'. Between the transducers 6" and 7", ultrasound pulses 5" are being sent in a straight line from the transmitting to the receiving transducer.

Fig. 4 shows three further examples of methods of ultrasound measurements where a transducer or a passive reflector is implanted into the body part 1. The combined transmitting/receiving transducer 6,7 is placed in acoustic contact with the skin surface 2, transmitting ultrasound pulses 5 into the body part 1. The same transducer 6, 7 then receives the echo from an implanted reflector 8. The implanted transmitting transducer 6' transmits ultrasound pulses 5' that are being received by the receiving transducer 7' at the skin surface 2. The implanted combined transmitting/ receiving transducer 6", 7" transmits ultrasound pulses 5" and receives echoes from the nearby tissue structures. In Fig. 4, only the echo from the skin surface 2 is illustrated, while echo from other tissue structures will also provide information about muscle contractions in the relevant region.

Different analysis methods can be used for estimating the relevant data from the ultrasound signals received by the receiving transducers 7, 7' and 7". Initially one performs a global and/or one or more local analyses of the reflected or transmitted signal from muscles and other tissue structures. This analysis is then utilized in order to estimate the motor intention. The analysis can be based on cross correlation between two or more ultrasound measurements, the individual measurements being distributed in time. The cross correlation analysis can be performed in one, two or three dimensions in order to measure muscle contractions in a corresponding number of dimensions. Alternatively, the analysis can be based on "optical flow" analysis, where two ore more temporally distributed ultrasound measurements are used to estimate the velocity field of the tissue in one, two or three dimensions. The estimated velocity field can be integrated to yield estimates of the tissue's rotation, translation and deformation in a corresponding number of dimensions. Furthermore, one or more muscle contractions can be classified and/or quantified by use of pattern recognition techniques, using figures calculated by the above mentioned analysis methods as input data to a pattern recognition unit.

The analysis can be based on measurements of tissue translation in one or more depths by integrating the phase change of the received signal (velocity measurement). Alternatively, the analysis can be based on measurement of the "time-of-flight" of the ultrasound pulse from the transmitting transducer 6, 6', 6''' to the receiving transducer 7, 7', 7".

In order to obtain more robust estimates of the user's motor intention, ultrasound measurements can be combined with measurements of other observable physiological signals and figures e.g. myoelectric signals (EMG) and/or nerve electric signals (ENG).

The present invention can also be combined with the user's interactions with mechanical, electrical and/or electromechanical input apparatus e.g. a harness with straps that measure shoulder movement, with switches or force sensors.

Estimation of muscle contraction can be performed statically or dynamically. Static estimation uses one or more of the above mentioned methods. Dynamic estimation can be conducted by use of a dynamic state estimator based on a mathematical model of the muscles' dynamic behaviour, where estimates from one or more of the above mentioned methods are uesd as input signals to the estimator.

As already mentioned, the transducers can be placed in a number of different ways. For example, they can be mounted in the prosthetic socket in such a way that they are in acoustic contact with the skin surface when the prosthesis is fitted on the body part. Gel, a special silicone pad or the like might be necessary to ensure good acoustic contact. Furthermore, the transducers can be mounted in a separate socket e.g. a "bracelet", that can be fitted on the body part without any rigid mechanical connection to the prosthetic socket, so that the force by which the transducer is pressed against the skin is independent of the forces acting between the prosthetic socket and the body part. The transducers can also be implanted into the body or have passive ultrasound reflectors implanted into the tissue to use as reference points in pulse-echo measurements.

More transducers can be used to obtain redundancy and hence quality improvements, allowing for automatic detection and correction of the transducer positions relative to the tissue, giving independent control of multiple prosthesis states, etc. An array probe consisting of a number of transducer elements can be used to send and measure in multiple directions or planes by appropriately delaying the signals associated with the individual array elements.

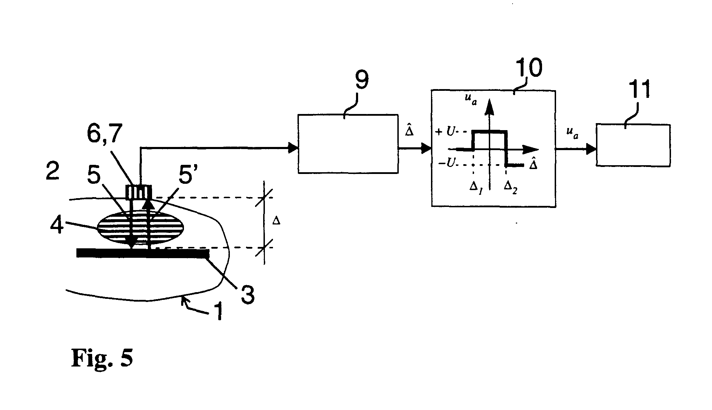

Fig. 5 shows the principal structure of a prosthesis control system for on/off control of a single prosthetic state. An ultrasound transducer 6, 7, acting both as a transmitter and a receiver, is placed on the skin surface 2 of a body part 1, with good acoustic contact, and such that a muscle 4 is located between the transducer and a bone 3. In this way both the transmitted ultrasound pulse 5 and the reflected ultrasound pulse 5' pass through the muscle 4. The signal from the transducer is fed into a unit 9 that performs signal processing and estimation. The unit 9 calculates, based on the observed signal, an estimate of the distance between the skin surface 2 and the bone 3. A signal from the unit 9 is fed into a unit 10 that controls the voltage to the prosthesis motor 11, according to the following scheme:

$$
u_a = \begin{cases} 0V \text{ when } ( \hat{\Delta} < \Delta_1 ) \\ U \text{ when } ( \Delta_1 < \hat{\Delta} < \Delta_2 ) \\ - U \text{ when } ( \hat{\Delta} > \Delta_2 ) \end{cases}
$$

If the prosthesis motor is a permanent magnet DC motor, the final angular velocity of the relevant joint function will be proportional to the voltage applied to the motor. The system depicted in Fig. 5 thus will make the relevant protshesis joint stop if the muscle 4 is relaxed or only lightly contracted ($\Delta < \Delta_1$). A moderate contraction ($\Delta_1 < \Delta < \Delta_2$) will cause a constant velocity in one direction, while a strong contraction ($\Delta > \Delta_2$) will cause a constant velocity in the opposite direction.

The unit 10 can be replaced by a continuous function. Alternatively it can include one or more feedback paths from prosthesis states, allowing for a more advanced control scheme.

**Claims**

1.   A device for control of prostheses and other assistance devices, by use of tissue whose state is influenced by the central nervous system of a user, comprising at least one transducer (6, 7), arranged to sense the state of the tissue, this information being fed into a unit (9, 10) that performs signal processing and estimation of the user's motor intention, whereby the device is arranged to control the prosthesis in correspondence with this estimate, **characterized in that**

   the at least one ultrasound transducer (6, 7) is arranged to send ultrasound signals into the tissue,
   the at least one ultrasound transducer (6, 7) is arranged to receive the ultrasound signals modulated by the tissue, and
   the unit (9, 10) for signal processing and estimation is connected to the at least one ultrasound transducer (6, 7), and estimates the user's motor intention based on said received ultrasound signals.

2.   The device in accordance with claim 1, **characterized in that** one and the same ultrasound transducer (6,7) is arranged for both transmission and reception of ultrasound signals.

3.   The device in accordance with claim 1, **characterized in that** a first ultrasound transducer (6) is arranged for transmission of ultrasound signals and that a second ultrasound transducer (7) is arranged for reception of ultrasound signals.

4.   The device in accordance with any one of the claims 1-3, **characterized in that** the at least one ultrasound transducer (6,7) is arranged for sequentially transmitting and receiving ultrasound signals with constant or varying time intervals.

5.   The device in accordance with any one of the claims 1-3, **characterized in that** the at least one ultrasound transducer (6,7) is arranged for continuously transmitting and receiving ultrasound signals.

6. The device in accordance with any one of the claims 1-5,
**characterized in that** the at least one ultrasound transducer (6,7) is arranged for transmitting and receiving ultrasound signals in one or more directions.

7. The device in accordance with any one of the claims 1-5,
**characterized in that** the at least one ultrasound transducer (6,7) is arranged for transmitting and receiving ultrasound signals in a continous space of two or more dimensions.

8. The device in accordance with any one of the claims 1-7,
**characterized in that** the receiver transducer (7) is arranged for receiving ultrasound signals that are transmitted from the transmitter transducer (6) via scattering/reflection by tissue structures.

9. The device in accordance with any one of the claims 1-7,
**characterized in that** the receiver transducer (7) is arranged for receiving ultrasound signals reflected by a number of ultrasound reflectors (8) that are implanted in the body part.

10. The device in accordance with any one of the claims 1-7,
**characterized in that** the receiver transducer (7) is arranged for receiving ultrasound signals that are transmitted from the transmitter transducer (6) via direct transmission through the tissue.

11. The device in accordance with any one of the claims 1-10,
**characterized in that** the at least one ultrasound transducer (6, 7) is arranged externally to and in good acoustic contact with the body part.

12. The device in accordance with any one of the claims 1-10,
**characterized in that** the at least one ultrasound transducer (6, 7) is implanted in the body part.

13. The device in accordance with any one of the preceding claims,
**characterized in that** the unit (9, 10) for signal processing and estimation is arranged to estimate the user's motor intention by cross correlation of a number of temporally separated ultrasound measurements or sets of measurements.

14. The device in accordance with any one of the preceding claims,
**characterized in that** the unit (9, 10) for signal processing and estimation is arranged to estimate the user's muscle contractions and motor intention by "optical flow" analysis of a number of temporally separated ultrasound measurements or sets of measurements.

15. The device in accordance with any one of the preceding claims,
**characterized in that** the unit (9, 10) for signal processing and estimation is arranged to combine ultrasound measurements with measurements of other observable physiological states of signals, such as myoelectric signals (EMG), nerve electric signals (ENG), or the user's interactions with mechanical, electric and/or electromechanical input devices.

16. The device in accordance with any one of the preceding claims.
**characterized in that** the unit (9, 10) for signal processing and estimation is arranged to estimate the user's muscle contractions and motor intention by use of a pattern recognition technique, where the input signals to the pattern recognition unit are generated by use of one or more of the techniques described in the preceding claims.

**Patentansprüche**

1. Gerät zur Steuerung von Prothesen und anderen Hilfsgeräten mittels eines Gewebes, dessen Zustand vom Zentralnervensystem eines Benutzers beeinflusst wird, das mindestens einen Messwertgeber (6, 7) aufweist, der zur Erfassung des Zustands des Gewebes ausgeführt ist, wobei diese Information in eine Einheit (9, 10) eingespeist wird, die eine Signalverarbeitung und eine Schätzung der motorischen Absicht des Benutzers vornimmt, wodurch das Gerät so arbeitet, dass es die Prothese entsprechend dieser Schätzung steuert,
**dadurch gekennzeichnet, dass**

der mindestens eine Ultraschall-Messwertgeber (6, 7) ausgeführt ist, um Ultraschallsignale in das Gewebe zu senden,

der mindestens eine Ultraschall-Messwertgeber (6, 7) ausgeführt ist, um vom Gewebe modulierte Ultraschallsignale zu empfangen, und

die Einheit (9, 10) zur Signalverarbeitung und Schätzung mit mindestens einem Ultraschall-Messwertgeber (6, 7) verbunden ist und die motorische Absicht des Benutzers auf Basis dieser empfangenen Ultraschallsignale schätzt.

2. Gerät nach Anspruch 1,
**dadurch gekennzeichnet, dass** ein und derselbe Ultraschall-Messwertgeber (6, 7) sowohl für Übertragung als auch Empfang von Ultraschallsignalen ausgeführt ist.

3. Gerät nach Anspruch 1,
**dadurch gekennzeichnet, dass** ein erster Ultraschall-Messwertgeber (6) für die Übertragung von Ultraschallsignalen und ein zweiter Ultraschall-Messwertgeber (7) für den Empfang von Ultraschallsignalen ausgeführt ist.

4. Gerät nach einem der Ansprüche 1 bis 3,
**dadurch gekennzeichnet, dass** der mindestens eine Ultraschall-Messwertgeber (6, 7) zum sequentiellen Übertragen und Empfangen von Ultraschallsignalen mit konstanten oder variierenden Zeitintervallen ausgeführt ist.

5. Gerät nach einem der Ansprüche 1 bis 3,
**dadurch gekennzeichnet, dass** der mindestens eine Ultraschall-Messwertgeber (6, 7) zum kontinuierlichen Übertragen und Empfangen von Ultraschallsignalen ausgeführt ist.

6. Gerät nach einem der Ansprüche 1 bis 5,
**dadurch gekennzeichnet, dass** der mindestens eine Ultraschall-Messwertgeber (6, 7) zum Übertragen und Empfangen von Ultraschallsignalen in einer oder mehreren Richtungen ausgeführt ist.

7. Gerät nach einem der Ansprüche 1 bis 5,
**dadurch gekennzeichnet, dass** der mindestens eine Ultraschall-Messwertgeber (6, 7) zum Übertragen und Empfangen von Ultraschallsignalen in einem kontinuierlichen Raum mit zwei oder mehr Dimensionen ausgeführt ist.

8. Gerät nach einem der Ansprüche 1 bis 7,
**dadurch gekennzeichnet, dass** der Empfänger-Messwertgeber (7) zum Empfangen von Ultraschallsignalen ausgeführt ist, die vom Sender-Messwertgeber (6) über Streuung/Reflexion durch Gewebestrukturen übertragen werden.

9. Gerät nach einem der Ansprüche 1 bis 7,
**dadurch gekennzeichnet, dass** der Empfänger-Messwertgeber (7) zum Empfangen von Ultraschallsignalen ausgeführt ist, die von einer Reihe Ultraschallreflektoren (8) reflektiert werden, die im Körperteil implantiert sind.

10. Gerät nach einem der Ansprüche 1 bis 7,
**dadurch gekennzeichnet, dass** der Empfänger-Messwertgeber (7) zum Empfangen von Ultraschallsignalen ausgeführt ist, die vom Sender-Messwertgeber (6) über direkte Übertragung durch das Gewebe übertragen werden.

11. Gerät nach einem der Ansprüche 1 bis 10,
**dadurch gekennzeichnet, dass** der mindestens eine Ultraschall-Messwertgeber (6, 7) außerhalb des und in gutem akustischen Kontakt mit dem Körperteil(s) angeordnet ist.

12. Gerät nach einem der Ansprüche 1 bis 10,
**dadurch gekennzeichnet, dass** der mindestens eine Ultraschall-Messwertgeber (6, 7) im Körperteil implantiert ist.

13. Gerät nach einem der vorstehenden Ansprüche,
**dadurch gekennzeichnet, dass** die Einheit (9, 10) zur Signalverarbeitung und Schätzung zur Schätzung der motorischen Absicht des Benutzers durch Querkorrelation einer Reihe zeitlich getrennter Ultraschallmessungen oder Mengen von Messungen ausgeführt ist.

**14.** Gerät nach einem der vorstehenden Ansprüche,
**dadurch gekennzeichnet, dass** die Einheit (9, 10) zur Signalverarbeitung und Schätzung zur Schätzung der Muskelkontraktionen und der motorischen Absicht des Benutzers durch eine "optische Fluss"-Analyse einer Reihe zeitlich getrennter Ultraschallmessungen oder Mengen von Messungen ausgeführt ist.

**15.** Gerät nach einem der vorstehenden Ansprüche,
**dadurch gekennzeichnet, dass** die Einheit (9, 10) zur Signalverarbeitung und Schätzung zur Kombination von Ultraschallmessungen mit anderen beobachtbaren physiologischen Signalzuständen wie myoelektrische Signale (EMG), nervenelektrische Signale (ENG) oder die Wechselwirkungen des Benutzers mit mechanischen, elektrischen und/oder elektromechanischen Eingabegeräten ausgeführt ist.

**16.** Gerät nach einem der vorstehenden Ansprüche,
**dadurch gekennzeichnet, dass** die Einheit (9, 10) zur Signalverarbeitung und Schätzung zur Schätzung der Muskelkontraktionen und der motorischen Absicht des Benutzers durch Anwendung einer Mustererkennungstechnik, bei der die Eingangssignale zur Mustererkennungseinheit durch Anwendung einer oder mehrerer der in den vorstehenden Ansprüchen beschriebenen Techniken generiert werden, ausgeführt ist.

**Revendications**

**1.** Dispositif pour commander des prothèses et autres dispositifs d'assistance, par utilisation de tissu dont l'état est influencé par le système nerveux central d'un utilisateur, comprenant au moins un transducteur (6, 7), agencé pour détecter l'état du tissu, ces informations étant fournies à une unité (9, 10) qui réalise le traitement et l'estimation des signaux de l'intention motrice de l'utilisateur, grâce à quoi le dispositif est agencé pour commander la prothèse en correspondance avec cette estimation,
**caractérisé en ce que** :

le transducteur à ultrasons (6, 7) est agencé pour envoyer des signaux ultrasonores dans le tissu,
le transducteur à ultrasons (6, 7) est agencé pour recevoir des signaux ultrasonores modulés par le tissu, et
l'unité (9, 10) pour le traitement et l'estimation des signaux est reliée au transducteur à ultrasons (6, 7), et estime l'intention motrice de l'utilisateur en se basant sur lesdits signaux ultrasonores reçus.

**2.** Dispositif selon la revendication 1,
**caractérisé en ce qu'**un et même transducteur à ultrasons (6, 7) est agencé pour à la fois transmettre et recevoir des signaux ultrasonores.

**3.** Dispositif selon la revendication 1,
**caractérisé en ce qu'**un premier transducteur à ultrasons (6) est agencé pour transmettre des signaux ultrasonores et **en ce qu'**un second transducteur à ultrasons (7) est agencé pour recevoir des signaux ultrasonores.

**4.** Dispositif selon l'une quelconque des revendications 1 - 3,
**caractérisé en ce que** ledit transducteur à ultrasons (6, 7) est agencé pour transmettre et recevoir séquentiellement des signaux ultrasonores avec des intervalles de temps constants ou variables.

**5.** Dispositif selon l'une quelconque des revendications 1 - 3,
**caractérisé en ce que** le transducteur à ultrasons (6, 7) est agencé pour transmettre et recevoir de façon continue des signaux ultrasonores.

**6.** Dispositif selon l'une quelconque des revendications 1 - 5,
**caractérisé en ce que** le transducteur à ultrasons (6, 7) est agencé pour transmettre et recevoir des signaux ultrasonores dans une ou plusieurs directions.

**7.** Dispositif selon l'une quelconque des revendications 1 - 5,
**caractérisé en ce que** le transducteur à ultrasons (6, 7) est agencé pour transmettre et recevoir des signaux ultrasonores dans un espace continu de deux ou plusieurs dimensions.

**8.** Dispositif selon l'une quelconque des revendications 1 - 7,
**caractérisé en ce que** le transducteur de réception (7) est agencé pour recevoir des signaux ultrasonores qui sont transmis du transducteur de transmission (6) via diffusion/réflexion par les structures tissulaires.

9. Dispositif selon l'une quelconque des revendications 1 - 7,
**caractérisé en ce que** le transducteur de réception (7) est agencé pour recevoir des signaux ultrasonores réfléchis par une pluralité de réflecteurs ultrasonores (8) qui sont implantés dans la partie corporelle.

10. Dispositif selon l'une quelconque des revendications 1 - 7,
**caractérisé en ce que** le transducteur de réception (7) est agencé pour recevoir des signaux ultrasonores qui sont transmis du transducteur de transmission (6) via une transmission directe à travers le tissu.

11. Dispositif selon l'une quelconque des revendications 1 - 10,
**caractérisé en ce que** le transducteur à ultrasons (6, 7) est agencé extérieurement à et en contact acoustique correct avec la partie corporelle.

12. Dispositif selon l'une quelconque des revendications 1 - 10,
**caractérisé en ce que** le transducteur à ultrasons (6, 7) est implanté dans la partie corporelle.

13. Dispositif selon l'une quelconque des revendications précédentes,
**caractérisé en ce que** l'unité (9, 10) pour le traitement et l'estimation des signaux est agencée pour estimer l'intention motrice de l'utilisateur par corrélation croisée d'une pluralité de mesures ou de séries de mesures ultrasonores temporellement séparées.

14. Dispositif selon l'une quelconque des revendications précédentes,
**caractérisé en ce que** l'unité (9, 10) pour le traitement et l'estimation des signaux est agencée pour estimer les contractions musculaires de l'utilisateur et l'intention motrice par analyse de "flux optique" d'une pluralité de mesures ou de séries de mesures ultrasonores temporellement séparées.

15. Dispositif selon l'une quelconque des revendications précédentes,
**caractérisé en ce que** l'unité (9, 10) pour le traitement et l'estimation des signaux est agencée pour combiner des mesures ultrasonores avec des mesures d'autres états physiologiques observables de signaux, tels que des signaux myoélectriques (EMG), des signaux électriques nerveux (ENG), ou les interactions de l'utilisateur avec des dispositifs d'entrée mécaniques, électriques et/ou électromécaniques.

16. Dispositif selon l'une quelconque des revendications précédentes,
**caractérisé en ce que** l'unité (9, 10) pour le traitement et l'estimation des signaux est agencée pour estimer l'intention motrice et les contractions musculaires de l'utilisateur par l'utilisation d'une technique de reconnaissance de forme, où les signaux d'entrée vers l'unité de reconnaissance de forme sont engendrés par l'utilisation d'une ou de plusieurs techniques décrites dans les revendications précédentes.

EP 0 865 262 B1

Fig. 1a

Fig. 1b

Fig. 2

**Fig. 3**

**Fig. 4**

**Fig. 5**